# EUROPÄISCHE PATENTSCHRIFT

(11) **EP 2 299 980 B1**
(45) Veröffentlichungstag und Bekanntmachung des Hinweises auf die Patenterteilung: **14.06.2017**
(21) Anmeldenummer: 09777154.7
(22) Anmeldetag: 13.07.2009
(51) Int. Cl.: A61K 9/16

(54) **SILICIUMDIOXID-NANOPARTIKEL UND DEREN VERWENDUNG ZUR VAKZINIERUNG**
SILICON DIOXIDE NANOPARTICLES AND THEIR USE FOR VACCINATION
NANOPARTICULES DE DIOXYDE DE SILICIUM ET LEUR UTILISATION POUR DES VACCINS

(30) Priorität: 15.07.2008 DE 102008033175
(43) Veröffentlichungstag der Anmeldung: 30.03.2011
(73) Patentinhaber: Merck Patent GmbH, 64293 Darmstadt (DE)
(72) Erfinder: WEIGANDT, Markus, 68259 Mannheim (DE); HANEFELD, Andrea, 69115 Heidelberg (DE); KUEBELBECK, Armin, 64625 Bensheim (DE); LARBIG, Gregor, 63571 Gelnhausen (DE)
(86) Internationale Anmeldenummer: PCT/EP2009/005078
(87) Internationale Veröffentlichungsnummer: WO 2010/006753

(56) Entgegenhaltungen:
- WO-A-95/29701
- WO-A-2007/030901
- JAIN T K ET AL: "Nanometer silica particles encapsulating active compounds: A novel ceramic drug carrier" JOURNAL OF THE AMERICAN CHEMICAL SOCIETY 19981104 US, Bd. 120, Nr. 43, 4. November 1998 (1998-11-04), Seiten 11092-11095, XP002562317 ISSN: 0002-7863
- KREUTER J ED - POWELL M Y ET AL: "NANOPARTICLES AS ADJUVANTS FOR VACCINES" VACCINE DESIGN. SUBUNIT AND ADJUVANT APPROACH; [PHARMACEUTICAL BIOTECHNOLOGY], NEW YORK, PLENUM PRESS, US, Bd. VOL. 6, 1. Januar 1995 (1995-01-01), Seiten 463-472, XP001006942 ISBN: 978-0-306-44867-6
- CHEN J-F ET AL: "Preparation and characterization of porous hollow silica nanoparticles for drug delivery application" BIOMATERIALS, ELSEVIER SCIENCE PUBLISHERS BV., BARKING, GB, Bd. 25, Nr. 4, 1. Februar 2004 (2004-02-01), Seiten 723-727, XP004471063 ISSN: 0142-9612
- REDDY SAI T ET AL: "Exploiting lymphatic transport and complement activation in nanoparticle vaccines" NATURE BIOTECHNOLOGY, Bd. 25, Nr. 10, Oktober 2007 (2007-10), Seiten 1159-1164, XP002568453 ISSN: 1087-0156
- VALLHOV HELEN ET AL: "Mesoporous silica particles induce size dependent effects on human dendritic cells." NANO LETTERS DEC 2007, Bd. 7, Nr. 12, Dezember 2007 (2007-12), Seiten 3576-3582, XP002562318 ISSN: 1530-6984
- BREITINGER H-G: "Synthesis of silica-bound amylose by phosphorolytic elongation of immobilised maltoheptaosyl hydrazides" TETRAHEDRON LETTERS, ELSEVIER, AMSTERDAM, Bd. 43, Nr. 35, 26. August 2002 (2002-08-26), Seiten 6127-6131, XP004373255 ISSN: 0040-4039

## Beschreibung

Die Erfindung betrifft ultrakleine, monodisperse Nanopartikel aus Siliciumdioxid, an deren Oberfläche mindestens ein Antigen befestigt ist, zur Zielsteuerung von Antigenen an Antigen-präsentierende Zellen und zur Aktivierung des Immunsystems sowie als Adjuvans, wobei die Effizienz von Zielsteuerung, Immunaktivierung und adjuvanter Wirkung über die Partikelcharakteristika gemäß der Ansprüche eingestellt wird. Die Nanopartikel können zur Immunprophylaxe oder Immuntherapie von Krebs sowie zur aktiven Immunisierung eines Säugers verwendet werden.

Die Gesundheit eines tierischen oder menschlichen Organismus hängt unter anderem davon ab, inwieweit der Organismus sich gegen pathogene Agenzien aus seiner Umwelt schützen kann bzw. inwieweit der Organismus verändertes körpereigenes Material erkennen und eliminieren kann. Das Immunsystem des menschlichen oder tierischen Körpers, das diese Funktionen erfüllt, lässt sich in zwei funktionelle Bereiche unterteilen: das angeborene und das erworbene Immunsystem. Die angeborene Immunität ist die erste Verteidigungslinie gegen Infektionen und die meisten potentiellen Krankheitserreger werden unschädlich gemacht, bevor sie beispielsweise eine erkennbare Infektion verursachen können. Das erworbene Immunsystem reagiert auf als Antigene bezeichnete Oberflächenstrukturen des eindringenden Organismus oder des veränderten körpereigenen Materials.

Es gibt zwei Typen erworbener Immunreaktionen: die humorale Immunreaktion und die zellvermittelte Immunreaktion. Bei der humoralen Immunreaktion binden in den Körperflüssigkeiten vorhandene Antikörper an Antigene und leiten deren Inaktivierung ein. Bei der zellvermittelten Immunreaktion werden T-Zellen aktiv, die andere Zellen zerstören können. Wenn beispielsweise mit einer Krankheit im Zusammenhang stehende Proteine in einer Zelle vorhanden sind, werden sie innerhalb der Zelle proteolytisch zu Peptiden fragmentiert. Danach binden spezifische Zellproteine an die so entstandenen Fragmente des Proteins oder Antigens und transportieren diese an die Oberfläche der Zelle, wo sie den molekularen Abwehrmechanismen, insbesondere T-Zellen des Körpers präsentiert werden.

Die Moleküle, die die Peptide an die Zelloberfläche transportieren und dort präsentieren, werden als Proteine des Haupthistokompatibilitätskomplexes (MHC) bezeichnet. Die Bedeutung der MHC-Proteine besteht auch darin, dass sie T-Zellen ermöglichen, eigene Antigene von nicht eigenen Antigenen zu unterscheiden. Die Kenntnis der Sequenz eines solchen nicht eigenen Peptides ermöglicht es, das Immunsystem gegen erkrankte Zellen zu manipulieren, z.B. unter Verwendung von Peptidimpfstoffen.

Technologien zur Präsentation von proteinogenen oder peptidischen Antigenen im Rahmen von Impfstoffen müssen zwei grundlegende Aufgaben erfüllen: den effizienten Transport des Antigens zu dendritischen Zellen und deren nachfolgende Aktivierung zum Hervorbringen einer erworbenen Immunantwort. Die gegenwärtige Impfstoffentwicklung fokussiert auf molekulare Strategien, die periphere dendritische Zellen, wie z.B. in der Haut oder Muskeln, als Ziel adressieren. Insbesondere durch Antikörper, die spezifisch für Zelloberflächenrezeptoren der dendritischen Zellen sind und entweder mit Antigenen fusioniert oder auf Partikeloberflächen angelagert sind, werden die Antigene zu ihrem dendritischen Bestimmungsort dirigiert. Solch anspruchsvolle Konstruktionen der zellspezifischen Zielsteuerung sind jedoch nicht zwingend, wie u.a. Fifis et al. (2004) J Immunol. 173(5), 3148, zeigen konnten, die eine Immunantwort durch den Transport von Silber konjugierten Polystyrenkügelchen zu dendritischen Zellen hervorriefen.

Es ist des Weiteren in der Immunologie bekannt, Adjuvantien einzusetzen, um die Immunantwort auf eine gegebene Substanz unspezifisch zu steigern. Während also das Antigen die spezifische Immunantwort bewirkt, ist für die Stärke dieser Antwort im Wesentlichen das Adjuvans verantwortlich. Um eine erworbene Immunantwort hervorzurufen, ist der Einsatz von Adjuvantien zur Induktion der dendritischen Zellreifung unerlässlich. Dendritische Zellen reifen hierbei im Ergebnis molekularer Gefahrensignale, die über Signalwege der angeborenen Immunität agieren, wie z.B. Toll-ähnliche Rezeptoren (TLRs) oder entzündliche Cytokinrezeptoren. In WO 2004/108072 A2 ist beispielsweise ein Konjugat beschrieben, in dem die Immunantwort modifizierende Verbindungen, wie z.B. TLR-Agonisten, an einen metallischen Partikelträger gebunden sind, der außerdem mindestens einen Wirkstoff aufweist. Die Immunantwort modifizierende Verbindungen sind dabei als Adjuvantien für Impfstoffe aufzufassen, die zwar eine starke Aktivierung der cytotoxischen Lymphozyten hervorbringen, jedoch den Aufbau der Partikel und deren wirtschaftliche Herstellung verkomplizieren sowie mit einem erhöhten Toxizitätsrisiko und physiologischen Transportbeschränkungen einhergehen.

WO 2001/12221 A1 beschreibt für Siliciumdioxid einen intrinsischen Adjuvanseffekt in Kombination mit proteinogenen Antigenen, Zellen oder Zellfragmenten, der auf rauen Kanten und einer unregelmäßigen Gestalt beruht, infolgedessen das Durchdringen von Zellmembranen und die Modifikation von Oberflächenproteinen ermöglicht wird. WO 2007/030901 A1 und Vallhov et al. (2007), Nano Lett. 7(12), 3576, assoziieren den Adjuvanseffekt dagegen mit der Mesoporosität der Silicapartikel. Unabhängig von der zugrundeliegenden Ursache lehrt bereits EP 0 465 081 B1 eine Zubereitung, umfassend ein Kernteilchen aus Metall, Keramik (z.B. Siliciumdioxid) oder Polymer, einen die Oberfläche dieses Kernteilchens zumindest teilweise bedeckenden Überzug, der einen Grundzucker, einen modifizierten Zucker oder ein Oligonukleotid umfasst, und mindestens ein mit dem beschichteten Kernteilchen in Berührung stehendes virales Protein oder Peptid. Die Kernteilchen weisen einen Durchmesser von 10 bis 200 nm auf, agglomerieren aber zu größeren Partikeln, was sogar erwünscht ist, da sich dadurch eine Depotwirkung einstellt. Nachteilig ist, dass sich durch solche Agglomerationen weder pharmazeutisch stabile Suspensionen erzeugen lassen noch eine Sterilfiltrierbarkeit gegeben ist.

Der Erfindung liegt die Aufgabe zugrunde, die im Stand der Technik aufgezeigten Nachteile zu überwinden und Nanopartikel zu entwickeln, die von monodisperser Partikelgröße sind und eine effektive Anwendung in der Immunprophylaxe oder Immuntherapie ermöglichen, insbesondere als Impfstoffe, die die therapeutische Wirksamkeit bei gleichzeitiger Verringerung der Nebenwirkungen verbessern.

Die Aufgabe der Erfindung wird gemäß den unabhängigen Ansprüchen gelöst. Die Unteransprüche beinhalten bevorzugte Ausführungsformen. Erfindungsgemäß werden monodisperse Nanopartikel zur Verwendung als Impfstoff zur Zielsteuerung von Antigenen an Antigen-präsentierende Zellen, zur Aktivierung des Immunsystems und als Adjuvans bereitgestellt, die eine Matrix umfassen, die mehr als 50 % Siliciumdioxid umfasst, und die an ihrer Oberfläche eine oder mehrere chemische Gruppen als Linker zur Befestigung und kontrollierten Freisetzung eines Antigens aufweisen, wobei an den Linker mindestens ein Antigen ortsspezifisch und kovalent gebunden ist, und wobei die Nanopartikel eine Größe von 5 bis 50 nm haben. Die Partikelgröße ist dabei so zu interpretieren, dass keine statistische Verteilung über den gesamten Bereich zwischen 5 und 50 nm vorliegt, sondern eine definierte Teilchengröße innerhalb des vorgenannten Bereichs gewählt wird, von der die Standardabweichung maximal 15 % beträgt, vorzugsweise maximal 10 %. In einer Ausführungsform der vorliegenden Erfindung haben die Partikel eine Größe zwischen 10 und 30 nm, vorzugsweise zwischen 20 und 30 nm, besonders bevorzugt zwischen 13 und 29 nm, ganz besonders bevorzugt von 25 nm ± 10 %.

Überraschenderweise hat sich gezeigt, dass durch das Bereitstellen von Siliciumdioxid-Nanopartikeln in einem engen Größenbereich zwischen 5 und 50 nm die Effizienz der Antigen-Zielsteuerung an Antigen-präsentierende Zellen signifikant erhöht werden kann. Insbesondere werden nicht mehr vordergründig periphere dendritische Zellen, sondern jene die dendritischen Zellen der Lymphknoten angesteuert. Die erfindungsgemäßen Nanopartikel sind durch ihre Größe und Materialauswahl derart ausgestaltet, dass eine wirkungsvolle Induktion der Reifung dendritischer Zellen erfolgt. Diese Induktion geschieht insbesondere über die Aktivierung des Komplementsystems. Die erfindungsgemäßen Siliciumdioxid-Nanopartikel eröffnen damit völlig neue Möglichkeiten hinsichtlich der Zielsteuerung zu Lymphknoten mit hoher dendritischer Zelldichte und hinsichtlich des Wegs der dendritischen Zellreifung als Voraussetzung der T-Zell-Proliferation und Immunisierung. Bemerkenswerterweise kommt ein auf diesen Nanopartikeln basierender Impfstoff ohne die in der Vakzinierung sonst unerlässlichen Adjuvantien aus.

Es ist bisher lediglich aus US 6,086,881 bekannt, dass das Impfstoffmaterial ein großes Molekulargewicht aufweisen soll, das die Wahrscheinlichkeit für antigene Determinanten erhöht. Ebenso ist es erwünscht, das Impfstoffmaterial an Alum oder anderen Gele zu aggregieren oder zu adsorbieren, da es gewöhnlich effektiver wird betreffs Zellbindung und Stimulation von Zelloberflächenmolekülen sowie aufgrund der langsamen Desorptionsgeschwindigkeit das Antigen über längere Zeiträume im Gewebe gehalten wird. Es wird auch durch Vallhov et al. (2007), Nano Lett. 7(12), 3576, bestätigt, dass größere Partikel aus mesoporösem Siliciumdioxid einen größeren Einfluss auf humane dendritische Zellen, die aus Monocyten abgeleitet sind, haben. Darüber hinaus werden im Stand der Technik gemäß WO 2008/019366 A2 Antigen-Silica-Konjugate zur Zielsteuerung an Antigen-präsentierende Zellen beschrieben, für die eine Partikelgröße von 0,3 bis 20 µm als notwendige Voraussetzung für die Phagozytose angesehen wird. Im Gegensatz dazu enthüllt die vorliegende Erfindung, dass gerade Siliciumdioxid-Nanopartikel in einem definierten engen Größenbereich von 5 bis 50 nm zur passiven Zielsteuerung an Antigen-präsentierende Zellen und zur Komplementaktivierung befähigt sind.

Unter einer "Antigen-präsentierenden Zelle" wird im Sinne der Erfindung jede Zelle verstanden, die zur Präsentation von Antigenen gegenüber einer T-Zelle induziert werden kann, wobei auch Vorläuferzellen eingeschlossen sind, die zu Antigen-präsentierenden Zellen ausdifferenziert und aktiviert werden können. Antigen-präsentierende Zellen beinhalten dendritische Zellen, Langerhans-Zellen, PBMCs, Makrophagen, B-Lymphozyten oder andere aktivierte oder modifizierte Zelltypen, wie z.B. Epithelzellen, Fibroblasten und Endothelzellen, die MHC-Moleküle auf ihren Zelloberflächen exprimieren, vorzugsweise dendritische Zellen, besonders bevorzugt dendritische Zellen der Lymphknoten. Vorläufer von Antigen-präsentierenden Zellen beinhalten CD34⁺-Zellen, Monocyten, Fibroblasten und Endothelzellen.

Erfindungsgemäß umfasst die partikuläre Bindematrix Siliciumdioxid zu mehr als 50 %. Der Bindematrix können also auch weitere Komponenten zugemischt sein, wobei Siliciumdioxid in einem Mehrkomponentensystem den höchsten Anteil zeigt.

Beispiele für andere Komponenten sind Metalle, Metallderivate, Metalloxide, Polymere, Organosilane, andere Keramiken oder Glas. Es ist bevorzugt, dass die Matrix zu mindestens 80 % Siliciumdioxid umfasst, besonders bevorzugt mindestens 90 %. In einer ganz besonders bevorzugten Ausführungsform der erfindungsgemäßen Nanopartikel umfasst die Matrix Siliciumdioxid, das im Wesentlichen rein ist, d.h. lediglich die im Zuge des Herstellungsverfahrens zu erwartenden Verunreinigungen aufweist. In einer höchst bevorzugten Ausführungsform der Erfindung besteht die partikuläre Bindematrix aus Siliciumdioxid.

Zur Präparation der Partikel bietet sich u.a. die klassische Stöber-Synthese an, worin über die Hydrolyse von Tetraethoxysilan (TEOS) in wässrig-alkoholischammoniakalischem Medium monodisperses nanoskaliges Siliciumdioxid definierter Größe hergestellt werden kann (J. Colloid Interface Sci. 1968, 26, 62). Die Erfinder konnten überraschend zeigen, dass die Stabilität der Nanopartikel trotz Oberflächenfunktionalisierung bestehen bleibt, infolgedessen monodisperse Partikel erhalten werden, die nicht zur Agglomeration neigen. Bevorzugt sind folglich Nanopartikel, die nach einem Verfahren mit den folgenden Schritten hergestellt werden:
(a) Hydrolytische Polykondensation von Tetraalkoxysilanen und/oder Organotrialkoxysilanen in einem Medium, das Wasser, mindestens einen Lösungsvermittler und mindestens ein Amin oder Ammoniak umfasst, wobei zunächst ein Sol von Primärteilchen erzeugt wird und anschließend derart, dass eine weitere Keimbildung verhindert wird, durch kontinuierliches, nach Maßnahme des Abreagierens kontrolliertes Zudosieren von entsprechendem Silan die erhaltenen Nanopartikel auf die gewünschte Teilchengröße in einem Bereich von 5 bis 50 nm gebracht werden, von der die Standardabweichung maximal 15 % beträgt, und
(b) Befestigen eines Antigens an einer Oberflächenfunktionalität der Nanopartikel, wobei die Nanopartikel an ihrer Oberfläche eine oder mehrere chemische Gruppen als Linker zur Befestigung und kontrollierten Freisetzung des Antigens aufweisen, wobei an den Linker mindestens ein Antigen ortsspezifisch und kovalent gebunden ist.

Sofern Ammoniak Bestandteil des Medium ist, wird als Lösungsvermittler insbesondere Alkohol verwendet, so dass die Reaktion in einem wässrig-alkoholisch-ammonikalischen Medium abläuft, wobei man hoch monodisperse Partikel erhält, deren Standardabweichung vom mittleren Teilchendurchmesser nicht mehr als 10 % beträgt. Die Erfinder haben nun überraschend gefunden, dass mit dem Verfahren sogar Teilchendurchmesser unter 50 nm mit den gewünschten monodispersen Eigenschaften zu realisieren sind. Der Schritt (a) des Verfahrens ist in EP 0 216 278 B1 sowie WO 2005/085135 A1 detailliert beschrieben. Vorzugsweise wird mindestens ein Amin im Medium verwendet.

Die Siliciumdioxid-Matrix der erfindungsgemäßen Nanopartikel kann sowohl porös als auch unporös sein. Die Porosität hängt wesentlich vom Herstellungsverfahren ab. Mit Blick auf die Synthese nach EP 0 216 278 B1 werden insbesondere nicht-poröse Partikel erhalten. Innerhalb des Eingangsbereichs von 5 bis 50 nm ist eine bevorzugte Partikelgröße für nicht-poröse Nanopartikel zwischen 10 und 30 nm, während die bevorzugte Partikelgröße für poröse Partikel 10 bis 40 nm beträgt. Bevorzugte Partikel der Erfindung sind solide.

Im Zusammenhang mit der vorliegenden Erfindung wird unter einem "Nanopartikel" eine partikuläre Bindematrix verstanden, die an ihrer Oberfläche Funktionalitäten aufweist, die als Erkennungsstellen für letztlich zu bindende Antigene fungieren. Die Oberfläche umfasst dabei sämtliche Flächen, d.h. neben der äußeren Oberfläche auch die innere Fläche von Hohlräumen (Poren) im Partikel. In einer erfindungsgemäßen Ausgestaltung kann das Antigen folglich in die Partikel aufgenommen werden, was die Porosität der Siliciumdioxid-Matrix voraussetzt.

Die Oberflächenfunktionalität kann sowohl aus einer oder mehreren chemischen Gruppen bestehen, die wiederum identisch oder verschieden sein können, wobei die Gruppen in ihrer Eigenschaft als Linker eine spezifische Befestigung von Nanopartikel und Antigen ermöglichen.

In der vorliegenden Erfindung ist das Antigen kovalent am Nanopartikel gebunden. Die kovalente Bindung kann indirekt erfolgen. Die indirekte Methode der kovalenten Verknüpfung nutzt einen Linker oder Tag, über den das Antigen ortsspezifisch an die Partikel gebunden und kontrolliert wieder freigesetzt wird. Tags für die ortsspezifische Konjugation sind im Stand der Technik bekannt, wie z.B. SNAP-Tag, HaloTag, C-terminaler LPXTG-Tag, Biotin-Akzeptor-Peptid, PCP oder ybbR-Tag, und werden u.a. in WO 2008/019366 A2 beschrieben.

In einer bevorzugten Ausführungsform wird die Oberflächenfunktionalität durch einen labilen Linker repräsentiert, besonders bevorzugt durch einen Hydrazon-Linker, Disulfid-Linker oder eine enzymatisch leicht zugängliche Peptidsequenz. Bei einem ersten klinischen Kandidaten ist Doxorubicin über eine säurelabile Hydrazon-Bindung als Sollbruchstelle mit dem Polymer verbunden (Angew. Chem. 2006, 118, 1218). Die Makromoleküle werden durch Endozytose in die Zelle aufgenommen, währenddessen es zu einem deutlichen Abfall im pH-Wert vom physiologischen Wert im Extrazellularraum (pH 7,2 - 7,4) auf pH 6,5 - 6 im Endosom und auf pH 4 in primären und sekundären Lysosomen kommt. Sinkt der pH-Wert in Folge der zellulären Aufnahme unterhalb von 6, zerfällt die Hydrazon-Verknüpfung und der Wirkstoff wird vom polymeren Träger gelöst. Dem Fachmann sind weitere spaltbare Linker bekannt, die sich im Sinne der Erfindung eignen und im weiteren Verlauf der Spezifikation beschrieben werden.

In einer weiteren bevorzugten Ausführungsform ist die Oberflächenfunktionalität aus der Gruppe der Alkoxysilane ausgewählt. Es ist hierbei besonders bevorzugt, dass es sich um eine endständige, reaktive Thiol-Gruppe handelt. Alkoxysilane sind sowohl zum Befestigen des Antigens als auch weiterer Liganden anderer Funktionen einsetzbar, wobei das Befestigen letzterer mit diesem stabilen Linker präferiert ist. Geeignete Alkoxysilane für die Zwecke der erfindungsgemäßen Nanopartikel können vom Fachmann routinemäßig ausgewählt werden.

Sofern die Oberfläche in Abhängigkeit vom gewählten Syntheseweg nicht bereits eine Funktionalisierung trägt, wird sie vor dem Befestigen des Antigens aufgebracht. Wenn die Nanopartikel durch hydrolytische Polykondensation gemäß dem oben genannten Verfahrensschritt (a) hergestellt werden, erfolgt die Funktionalisierung der Oberfläche nach Schritt (a) und vor Schritt (b). Viele der Siliciumatome auf der Partikelhülle tragen Hydroxylfunktionen, welche nach Standardmethoden mit einer Mehrzahl an kommerziell erhältlichen Trialkoxysilanen bzw. Trichlorsilanen reagieren können, so dass sich die Partikel auf einfachem Wege verschiedenartig funktionalisieren lassen (J. Liq. Chrom. & rel. Technol. 1996, 19, 2723). Sofern die angestrebten Anwendungen bzw. die gewünschten Eigenschaften der nanoskaligen Siliciumdioxid-Partikel eine höhere chemische Komplexität erfordern, werden ausgeklügelte Mehrstufensynthesen angewendet.

Unter einem "Antigen" ist vorliegend eine Struktur zu verstehen, die in der Lage ist, eine zelluläre oder tierische Immunantwort zu erzeugen. Es versteht sich, dass die Immunantwort in einem Tier alle Säuger, insbesondere den Menschen, einschließt. Antigene sind vorzugsweise proteinogen, d.h. es handelt sich um Proteine, Polypeptide, Peptide oder deren Fragmente, die wiederum von beliebiger Größe, Herkunft und Molekulargewicht sowie glycosiliert sein können, aber mindestens eine antigene Determinante oder ein antigenes Epitop beinhalten. Eine Erkennung durch das Immunsystem erfolgt insbesondere ab einer Mindestlänge von drei Aminosäuren. Die Proteine oder Peptide sind vorzugsweise ausgewählt aus der Gruppe der Cytokine, Rezeptoren, Lektine, Avidine, Lipoproteine, Glycoproteine, Oligopeptide, Peptid-Liganden und Peptid-Hormone. Antigene können auch Nukleinsäuren per se sein oder durch Nukleinsäuren kodiert werden, die nach dem Transport in den Kern von Antigen-präsentierenden Zellen in das proteinogene Antigen translatiert werden, welches auf MHC-Molekülen präsentiert wird. Die Nukleinsäuren sind einzel- und doppelsträngige DNA oder RNA sowie Oligonukleotide. Die Nukleinsäuren können auch Bestandteil von Komplexen oder Formulierungen sein, die aus Lipiden, Kohlenhydraten, Proteinen oder Peptiden bestehen. Weitere Antigene sind Polysaccharide, Polymere, niedermolekulare Substanzen mit einem Molekulargewicht von 50 bis 1000 Da, Viren, intakte prokaryotische oder eukaryotische Zellen oder zelluläre Fragmente.

In einer Ausführungsform der Erfindung hat das Antigen ein Molekulargewicht unter 500 kDa. Das Antigen ist vorzugsweise ein Krebs-Antigen. Solche Krebs-Antigene sind beispielsweise in WO 2008/019366 A2 offenbart. In einer besonders bevorzugten Ausführungsform ist das Krebs-Antigen ausgewählt aus der Gruppe umfassend New York Esophageal 1 Antigen (NY-ESO-I), MAGE-A1, MAGE-A2, MAGE-A3, MAGE-A4, MAGE-A6, MAGE-A8, MAGE-A10, MAGE-B, MAGE-C1, MAGE-C2, L Antigen (LAGE), SSX2, SSX4, SSX5, PRAME, Melan-A, Caspase-8, Tyrosinase, MAGF, PSA, CEA, HER2/neu, MUC-1, MART1, BCR-abl, p53, ras, myc, RB-1 und Survivin oder Epitope davon. In einer ganz besonders bevorzugten Ausführungsform der Erfindung ist das Krebs-Antigen Survivin oder dessen Epitope. Dieses Krebs-Antigen ist in WO 2007/039192 A2 beschrieben.

Die Nanopartikel können mehrfach funktionalisiert sein, was verschiedene chemische Gruppen (Oberflächenfunktionalitäten) und/oder verschiedene gebundene Moleküle (Funktionen) bedeutet. Es ist bevorzugt, dass sowohl die Oberflächenfunktionalitäten als auch die gebundenen Funktionen verschieden sind, wodurch eine spezifische, unabhängige Bindung der funktionalen Moleküle gegeben ist. Die Funktionen sind vorzugsweise ausgewählt aus der Gruppe von Antigen, Polyethylenglycol (PEG), Markierung und Adjuvans, wobei es sich versteht, dass das Antigen immer ausgewählt ist. Besonders bevorzugt sind Antigen und PEG und/oder Adjuvans vorhanden, ganz besonders bevorzugt Antigen, PEG und Adjuvans, wobei diese Funktionen adsorptiv und/oder kovalent gebunden sein können.

In einer Ausgestaltung der Erfindung wird die Markierung durch Lumineszenz, UV/VIS-Farbgebung, enzymatisch, elektrochemisch oder radioaktiv nachgewiesen. Vorzugsweise finden Fluoreszenzfarbstoffe oder radiaktive Markierungen Anwendung. Bei der Photolumineszenz oder Fluoreszenz erfolgt die Anregung durch Absorption von Photonen. Bevorzugte Fluorophore sind Bisbenzimidalole, Fluorescein, Acridinorange, Cy5, Cy3 oder Propidiumiodid. Die Auswertung geschieht visuell oder mit entsprechenden Messgeräten, z.B. im Fluoreszenzmikroskop, oder durch Durchflusscytometrie, z.B. im Cytofluorimeter. In einer besonders bevorzugten Ausgestaltung der Erfindung werden die Fluoreszenzfarbstoffe an 3-Aminopropyltriethoxysilan gebunden, wobei es sich bei Fluoresceinisothiocyanat um einen ganz besonders bevorzugten Fluoreszenzfarbstoff handelt.

Alternativ kann der Nachweis auch radioaktiv mit radioaktiven Isotopen erfolgen, vorzugsweise mit ³H, ¹⁴C, ³²P, ³³P, ³⁵S, ^{99m} Tc, ¹¹¹In oder ¹²⁵I, besonders bevorzugt mit ^{99m}Tc oder ¹¹¹In. Insbesondere werden Derivate der 1,4,7,10-Tetraazacyclododekan-N,N',N",N"'-Tetraessigsäure (DOTA) oder Diethylentriaminpentaessigsäure (DTPA), die über Klick-Chemie an die Nanopartikel gebunden sind, unmittelbar vor der Injektion mit den besonders bevorzugten Radioisotopen belegt. Bei der Sintillationszählung wird z.B. ein Molekülcocktail durch radioaktive γ-Strahlung angeregt. Die beim Übergang in den Grundzustand als Licht freigesetzte Energie wird durch einen Photoelektronenvervielfacher verstärkt und gezählt.

Damit haben die erfindungsgemäßen Nanopartikel auch Bedeutung als diagnostisches Werkzeug (z.B. in bildgebenden Verfahren) und/oder Forschungswerkzeug, das die Visualisierung der Zielsteuerung und Wirkstoffaufnahme ermöglicht.

In einer weiteren Ausgestaltung der Erfindung wird ein Antigen mit einer Markierung so kombiniert, dass innerhalb einer Partikelfraktion über die Markierung eine Zuordnung zum Antigen stattfinden kann. Das bedeutet, dass ein erstes Partikel bzw. eine Pluralität davon mit einem ersten Antigen und einer ersten Markierung versehen ist, während ein zweites Partikel bzw. eine Pluralität davon mit einem zweites Antigen und einer zweiten Markierung ausgestattet ist, etc., wobei sich sowohl die Antigene als auch die Markierungen jeweils untereinander unterscheiden. Die spezifische Kombination von Antigen und Markierung ist hierin folglich einmalig und bevorzugt und ermöglicht ein Mischen von Partikeln mit unterschiedlichen Antigenen und das parallele Verfolgen der Zielsteuerungseffizienz und/oder Immun-/Komplementaktivierung. Daraus resultiert eine Zeitersparnis in der Diagnostik gegenüber der sequentiellen Verabreichung. Es ist natürlich ebenso möglich, dass die Partikel mehrere Antigene und mehrere Markierungen tragen, deren Intensitäten variieren, so dass aus der Abmischung auf ein bestimmtes Antigen geschlossen werden kann. Bei der Markierung handelt es sich vorzugsweise um einen Fluoreszenzfarbstoff, der insbesondere an Silan gebunden ist.

Die Nanopartikel der Erfindung können des Weiteren als Kombinationen von Antigenen und Gefahrensignalen, wie z.B. TLRs oder Cytokinen, ausgeführt werden.

In noch einer weiteren Ausgestaltung der vorliegenden Erfindung ist die Oberfläche derart mehrfach funktionalisiert, dass eine Quervernetzung der Mehrfachfunktionalitäten ausgeschlossen ist.

Die vorherige Lehre der Erfindung und deren Ausführungsformen betreffend die Oberflächenfunktionalität zur Befestigung von Antigenen am Nanopartikel sind gültig und ohne Einschränkungen auf Mehrfachfunktionalitäten und/oder die Befestigung weiterer Funktionen am Nanopartikel anwendbar, sofern es sinnvoll erscheint.

Eine universelle Strategie zum Aufbau hochkomplexer Systeme stellt das von K. B. Sharpless (Angew. Chem. Int. Ed. 2001, 40, 2004) vorgestellte Konzept der Klick-Chemie dar. Es handelt sich dabei eher um eine Synthesephilosophie als um eine wissenschaftliche Disziplin, die vor allem von der Einfachheit und Effizienz in der Natur vorkommender Reaktionen inspiriert ist. Als Paradebeispiel für die Klick-Chemie stellte sich die 1,3-dipolare Cycloaddition von Aziden und terminalen Alkinen nach Huisgen heraus. Bei Anwesenheit von einwertigem Kupfer finden diese Reaktionen drastisch beschleunigt statt, verlaufen zudem regioselektiv, in sehr hohen Ausbeuten und unter Toleranz einer großen Bandbreite von funktionellen Gruppen. Ein weiterer Vorteil liegt in der Möglichkeit, die Synthese im wässrigen Milieu und bei Raumtemperatur durchzuführen, so dass man interessante Biomoleküle in einer Art Baukastenprinzip modular und breit anwendbar mit anderen Bausteinen verknüpfen kann. Es ist deshalb bevorzugt, unter Anwendung der Klick-Chemie die entsprechend funktionalisierten Siliciumdioxid-Partikel mit den vorgenannten Funktionen zu verknüpfen, insbesondere den Antigenen.

Eine Dispersion kann bereitgestellt werden, die die erfindungsgemäßen Nanopartikel umfasst. Die Nanopartikel können in einem beliebigen Lösungsmittel dispergiert werden, solange die Nanopartikel durch das Lösungsmittel weder chemisch angegriffen noch physikalisch verändert werden und umgekehrt, so dass die resultierende Nanodispersion stabil ist, insbesondere pharmazeutisch und physikalisch stabil. Die Dispersion ist speziell dadurch gekennzeichnet, dass die Nanopartikel monodispergiert und nicht aggregiert sind sowie nicht zur Sedimentation neigen, was in Sterilfiltrierbarkeit resultiert. Die vorherige Lehre der Erfindung und deren Ausführungsformen betreffend die monodispersen Nanopartikel sind gültig und ohne Einschränkungen auf die Dispersionen anwendbar, sofern es sinnvoll erscheint.

Ein Kit, der die erfindungsgemäßen Nanopartikel bzw. deren Dispersionen umfasst, kann auch einen Artikel beinhalten, der schriftliche Anweisungen enthält oder den Anwender auf schriftliche Anweisungen hinweist, die den Umgang mit den Nanopartikeln der Erfindung erläutern.

Ein pharmazeutisches Mittel, das die erfindungsgemäßen Nanopartikel bzw. deren Dispersionen umfasst, ist hierbei jedes Mittel, welches in der Prophylaxe, Therapie, Verlaufskontrolle oder Nachbehandlung von Patienten eingesetzt werden kann, die zumindest zeitweise eine pathogene Modifikation des Gesamtzustandes bzw. des Zustandes einzelner Teile des Patientenorganismus zeigen, insbesondere infolge von Infektionskrankheiten, septischem Schock, Tumoren, Krebs, Autoimmunerkrankungen, Allergien und chronischen oder akuten Entzündungsprozessen. So ist das pharmazeutische Mittel im Sinne der Erfindung ein Impfstoff (Vakzin). Der Impfstoff kann die Antigene, wie z.B. Peptide oder Nukleinsäuren, beispielsweise als ein pharmazeutisch akzeptables Salz umfassen. Hierbei kann es sich u.a. um Salze anorganischer Säuren, wie z.B. der Phosphorsäure, oder um Salze organischer Säuren handeln.

Zur Unterstützung der medizinischen Wirkung, d.h. insbesondere der Immunantwort, kann der Impfstoff in einer Ausführungsform der Erfindung auch weitere Wirkstoffe umfassen, wobei eine gleichzeitige oder aufeinander folgende Verabreichung denkbar ist. Die therapeutische Wirkung des erfindungsgemäßen Impfstoffs kann dann beispielsweise darin bestehen, dass durch die Aktivierung des Komplementsystems als erwünschter Nebeneffekt bestimmte Antitumor-Medikamente besser wirken oder durch die Verminderung der Dosis die Anzahl der Nebenwirkungen dieser Medikamente reduziert wird.

In einer bevorzugten Ausführungsform der Erfindung wird der erfindungsgemäße Impfstoff mit Chemotherapeutika kombiniert, die ausgewählt sind aus der Gruppe umfassend Cytokine, Chemokine, pro-apoptotische Mittel, Interferone, radioaktive Verbindungen oder Kombinationen davon. Es ist bevorzugt, dass das Chemotherapeutikum den Nukleinsäure- und/oder Proteinstoffwechsel, die Zellteilung, DNA-Replikation, Purin-, Pyrimidin- und/oder Aminosäure-Biosynthese, Genexpression, mRNA-Prozessierung, Proteinsynthese, Apoptose oder Kombinationen davon verändert, insbesondere abschwächt.

Zur Anregung der körpereigenen Abwehrkräfte oder der Stärkung des Immunsystems können in einer weiteren Ausführungsform der Erfindung mit dem vorliegenden Impfstoff auch Immunstimulantien gegeben werden, beispielsweise Interferone, wie z.B. IFN-α, IFN-β oder IFN-γ, Interleukine, wie z.B. IL-1, IL-2, IL-3, IL-4, IL-5, IL-6, IL-7, IL-8, IL-9, IL-10 oder IL-12, Tumor-Nekrose-Faktoren, wie z.B. TNF-α oder TNF-β, Erythropoietin, M-CSF, G-CSF, GM-CSF, CD2 oder ICAM. Auf diese Weise können die Proliferation, Entwicklung, Differenzierung oder Aktivierung von beispielsweise T-Lymphozyten, B-Lymphozyten, Monozyten, Makrophagen, neutrophilen Zellen, eosinophilen Zellen, Megakaryozyten und/oder Granulozyten stimuliert werden.

Um die protektive oder therapeutische Wirkung der erfindungsgemäßen immunogenen Nanopartikel zu erhöhen, können den Partikeln bzw. dem Impfstoff, der aus diesen hergestellt wird, pharmazeutisch verträgliche Adjuvantien zugesetzt werden. Jede Substanz, die mit den Antigenen gemäß der Erfindung eine Wirkung ermöglicht, verstärkt oder modifiziert, ist ein "Adjuvans". Bekannte Adjuvantien sind beispielsweise Aluminiumverbindungen, wie z.B. Aluminiumhydroxid oder Aluminiumphosphat, Saponine, wie z.B. QS 21, Muramyldipeptid oder Muramyltripeptid, Proteine, wie z.B. Gammainterferon oder TNF, MF 59, Phosphatdibylcholin, Squalen oder Polyole. Ebenfalls kann die Co-Applikation von Ei-Albumin in komplettem Freund'schen Adjuvans eine gesteigerte zellvermittelte Immunität hervorrufen und somit die Wirkung gebildeter neutralisierender Antikörper unterstützen. Des Weiteren kann DNA, die eine immunstimulatorische Eigenschaft hat, oder die ein Protein mit Adjuvanseffekt kodiert, wie z.B. ein Cytokin, parallel oder in einem Konstrukt appliziert werden. Aufgrund des intrinsischen Adjuvanseffekts der erfindungsgemäßen Nanopartikel auf Siliciumdioxid-Basis ist es jedoch vorliegend bevorzugt, keine weiteren Adjuvantien zu verwenden. Sofern der intrinsische Adjuvanseffekt in bestimmten Anwendungen sich als nicht ausreichend erweist, können natürlich ein oder mehrere Adjuvantien zusätzlich an den Nanopartikel befestigt werden, vorzugsweise nur ein Adjuvans. Die Art der Befestigung kann sowohl adsorptiv sein als auch in einer kovalenten Bindung bestehen. Bevorzugte adsorptiv zu bindende Adjuvantien umfassen Poloxamere und TLRs. Bevorzugte kovalent gebundene Adjuvantien umfassen kurzkettige Peptide, besonders bevorzugt Tuftsin oder Ovalbumin.

Das Einbringen des Impfstoffs in eine Zelle respektive einen Organismus kann auf jede Art und Weise geschehen, die es ermöglicht, dass die Antigen-präsentierenden Zellen mit den im Impfstoff enthaltenen Nanopartikeln bzw. Antigenen in Kontakt gebracht und durch Phagozytose in die Zellen aufgenommen werden, infolgedessen eine Immunantwort induziert wird. Der Impfstoff der vorliegenden Erfindung kann oral, transdermal, transmucosal, transurethal, vaginal, rektal, pulmonal, enteral und/oder parenteral angewendet werden. Bevorzugt ist die parenterale Anwendung des Impfstoffs. Es konnte vorliegend gezeigt werden, dass Siliciumdioxid in seiner Eigenschaft als Adjuvans keine negativen Auswirkungen auf den Lipidhaushalt hat, wie es für polymere Adjuvantien beobachtet wurde, die folglich für diese Applikationsart nicht zugelassen sind. Besonders bevorzugt ist eine direkte Injektion in den Körper. Die gewählte Art der Verabreichung richtet sich nach der Indikation, der zu verabreichenden Dosis, Individuums-spezifischen Parametern etc. Insbesondere ermöglichen die verschiedenen Arten der Verabreichung eine ortsspezifische Therapie, die Nebenwirkungen minimiert und die Wirkstoffdosis verringert. Ganz besonders bevorzugte Injektionen sind die intradermale, subkutane, intramuskuläre oder intravenöse Injektion. Die Applikation kann z.B. mit Hilfe so genannter Impfpistolen oder mittels Spritzen geschehen. Es ist auch möglich, die Substanz als Aerosol bereitzustellen, welches von dem Organismus, bevorzugt einem humanen Patienten, inhaliert wird.

Die Darreichungsformen des Impfstoffs werden mit den üblichen festen oder flüssigen Trägerstoffen und/oder Verdünnungsmitteln und den üblicherweise eingesetzten Hilfsstoffen entsprechend der gewünschten Applikationsart in einer geeigneten Dosierung und in an sich bekannter Weise hergestellt. Grundsätzlich können also pharmazeutisch annehmbare und dem Fachkundigen bekannte Exzipienten einen Teil der erfindungsgemäßen pharmazeutischen Zusammensetzung bilden, wobei die Menge des Exzipientenmaterials, die mit dem Wirkstoff kombiniert wird, um eine Einzeldosierung herzustellen, in Abhängigkeit vom zu behandelnden Individuum und der Art der Verabreichung variiert. Diese pharmazeutisch verträglichen Zusätze umfassen Salze, Puffer, Füllstoffe, Stabilisatoren, Komplexbildner, Antioxidantien, Lösungsmittel, Bindemittel, Gleitmittel. Tablettenüberzüge, Geschmacksstoffe, Farbstoffe, Konservierungsmittel, Stellmittel und dergleichen. Beispiele für solche Exzipienten sind Wasser, pflanzliche Öle, Benzylalkohole, Alkylenglycol, Polyethylenglycol, Glycerintriacetat, Gelatine, Kohlenhydrate, wie z.B. Lactose oder Stärke, Magnesiumstearat, Talk und Vaseline.

Die pharmazeutische Formulierung kann als Tablette, Filmtablette, Dragee, Lutschtablette, Kapsel, Pille, Pulver, Granulat, Sirup, Saft, Tropfen, Lösung, Dispersion, Suspension, Suppositor, Emulsion, Implantat, Creme, Gel, Salbe, Paste, Lotion, Serum, Öl, Spray, Aerosol, Kleber, Pflaster oder Verband vorliegen, wobei die Dispersion bevorzugt ist.

Als orale Darreichungsform werden vorzugsweise Tabletten, Filmtabletten, Dragees, Lutschtabletten, Kapseln, Pillen, Pulver, Granulate, Sirupe, Säfte, Tropfen, Lösungen, Dispersionen oder Suspensionen - auch als Depotform - zubereitet. Arzneiformen als Tabletten können beispielsweise durch Mischen des Wirkstoffes mit bekannten Hilfsstoffen, wie Dextrose, Zucker, Sorbit, Mannit, Polyvinylpyrrolidon, Sprengmitteln, wie Maisstärke oder Alginsäure, Bindemitteln, wie Stärke oder Gelatine, Gleitmitteln, wie Magnesiumstearat oder Talk, und/oder Mitteln, die einen Depoteffekt erzielen können, wie Carboxypolymethylen, Carboxymethylcellulose, Celluloseacetatphthalat oder Polyvinylacetat, erhalten werden. Die Tabletten können auch aus mehreren Schichten bestehen. Ebenso lassen sich Dragees durch Überziehen von analog zu den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker bereiten. Die Drageehülle kann dabei auch aus mehreren Schichten bestehen, wobei beispielsweise die oben genannten Hilfsstoffe verwendet werden. Die Kapseln können durch Mischen des Wirkstoffes mit Trägern, wie Milchzucker oder Sorbit, hergestellt werden, die dann in Kapseln eingebracht werden. Die Lösungen oder Dispersionen des Impfstoffs können zur Verbesserung des Geschmacks mit Stoffen, wie z.B. Saccharin, Cyclamat oder Zuckerarten, und/oder mit Aromastoffen, wie z.B. Vanillin oder Orangeextrakt, versetzt werden. Weiterhin können sie mit Suspendierhilfsstoffen, wie z.B. Natriumcarboxymethylcellulose, oder Konservierungsmitteln, wie z.B. p-Hydroxybenzoesäure, Phenol, Benzylalkohol, *m*-Kresol, Methylparaben, Propylparaben, Benzalkoniumchlorid oder Benzethoniumchlorid, vermischt werden.

Des Weiteren sind parenterale Arzneiformen, wie z.B. Suppositorien, Suspensionen, Emulsionen, Implantate oder Lösungen, in Betracht zu ziehen, vorzugsweise ölige oder wässrige Lösungen. Zur parenteralen Verabreichung kann das immunogene Konstrukt der Erfindung in einem physiologisch verträglichen Verdünnungsmittel gelöst oder suspendiert werden, wie z.B. Neutralfetten oder Polyethylenglykolen oder dessen Derivaten. Als bevorzugte Lösungsmittel werden häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnussöl, Baumwollsamenöl, Rizinusöl und Sesamöl.

Zur topischen Applikation des Impfstoffs wird dieser mit mindestens einem pharmazeutisch annehmbaren Trägerstoff, wie z.B. mikrokristalliner Cellulose, und ggf. weiteren Hilfsstoffen, wie z.B. Feuchtigkeitsspendern, zu auf die Haut auftragbaren festen Formulierungen, wie z.B. Cremes, Gelen, Salben, Pasten, Pulver oder Emulsionen bzw. zu auf die Haut auftragbaren flüssigen Formulierungen, wie z.B. Lösungen, Suspensionen, Lotionen, Seren, Ölen, Sprays oder Aerosole in üblicher Weise formuliert. Beispiele sind Lösungen in Alkoholen, wie z.B. Ethanol oder Isopropanol, Acetonitril, DMF, Dimethylacetamid, 1,2-Propandiol oder deren Mischungen untereinander und/oder mit Wasser. Als Trägersysteme für den Impfstoff können auch Liposomen dienen, die einen optimalen Transport in die Haut gewährleisten. Als topische Zubereitungen kommen auch transdermale Systeme in Betracht, wie z.B. Lösungen, Suspensionen, Cremes, Salben, Pulver, Gele, Emulsionen, Kleber, Pflaster oder Verbände, die die Nanopartikel zusammen mit einem Träger enthalten. Nützliche Träger können absorbierbare, pharmakologisch geeignete Lösungsmittel enthalten, um den Durchtritt der Nanopartikel durch die Haut zu unterstützen. Lösungsmittel, die eine gute Penetration in die Haut gewährleisten sind beispielsweise die Alkohole Phenylethanol-1, Glycerin, Ethanol oder deren Mischungen.

Vorzugsweise liegt der Impfstoff als Injektionslösung vor. Für die Herstellung der Injektionslösung können wässrige Medien, wie z.B. destilliertes Wasser oder physiologische Salzlösungen verwendet werden, wobei letztere saure und basische Additionssalze einschließen. Der Impfstoff kann auch als feste Zusammensetzung, beispielsweise im lyophilisierten Zustand vorliegen, und dann durch Zugabe eines auflösenden Mittels, wie z.B. destilliertes Wasser, vor der Verwendung bereitet werden. Die Grundlagen der Herstellung von Lyophilisaten sind dem Fachmann vertraut.

Die Konzentration der aktiven Nanopartikel in der Formulierung kann zwischen 0,1 bis 100 Gewichtsprozenten variieren. Entscheidend ist, dass die pharmazeutische Zusammensetzung als Wirkstoff eine effektive Menge der erfindungsgemäßen Nanopartikel bzw. deren Dispersion zusammen mit den pharmazeutisch verträglichen Hilfsstoffen umfasst. Die Begriffe "effektive Menge" oder "wirksame Dosis" werden hierin austauschbar verwendet und bezeichnen eine Menge des pharmazeutischen Wirkstoffs, die eine prophylaktisch oder therapeutisch relevante Wirkung auf eine Krankheit oder pathologische Veränderung hat. Eine "prophylaktische Wirkung" verhindert das Ausbrechen einer Krankheit oder sogar die Infektion mit einem Erreger nach dem Eindringen einzelner Vertreter derart, dass deren nachfolgende Ausbreitung stark verringert wird oder sie sogar vollständig inaktiviert werden. Eine "therapeutisch relevante Wirkung" befreit von einem oder mehreren Krankheitssymptomen oder führt zur teilweisen oder vollständigen Rückkehr eines oder mehrerer physiologischer oder biochemischer Parameter, die mit der Krankheit oder pathologischen Veränderung in Zusammenhang stehen oder ursächlich dafür sind, in den Normalzustand. Die jeweilige Dosis bzw. der Dosisbereich für die Gabe der erfindungsgemäßen Nanopartikel ist groß genug, um den gewünschten prophylaktischen oder therapeutischen Effekt der Induktion einer Immunantwort zu erreichen. Im Allgemeinen wird die Dosis mit dem Alter, der Konstitution und dem Geschlecht des Patienten variieren sowie die Schwere der Erkrankung berücksichtigen. Es versteht sich, dass die spezifische Dosis, Häufigkeit und Dauer der Verabreichung darüber hinaus von einer Vielzahl an Faktoren abhängen, wie z.B. der Zielsteuerungs- und Bindungsfähigkeit der Nanopartikel, Ernährungsgewohnheiten des zu behandelnden Individuums, Art der Verabreichung, Ausscheidungsrate und Kombination mit anderen Medikamenten. Die individuelle Dosis kann sowohl in Bezug auf die primäre Erkrankung als auch in Bezug auf das Eintreten eventueller Komplikationen eingestellt werden. Die exakte Dosis ist durch einen Fachmann mit bekannten Mitteln und Methoden feststellbar. Diese Lehre der Erfindung ist gültig und ohne Einschränkungen auf die pharmazeutische Zusammensetzung umfassend die erfindungsgemäßen Nanopartikel bzw. Dispersionen davon anwendbar, sofern es sinnvoll erscheint.

In einer Ausführungsform der Erfindung werden die Nanopartikel in einer Dosis von 0,01 mg bis 1 g pro Kilogramm Körpergewicht und pro Tag verabreicht. Vorzugsweise werden jedoch Dosen von 20 bis 60 mg pro Kilogramm Körpergewicht und pro Tag verabreicht. Die tägliche Dosierung liegt vorzugsweise zwischen 0,02 und 10 mg/kg Körpergewicht.

Die vorliegenden Nanopartikel bzw. Nanopartikel-Dispersionen sind zur prophylaktischen oder therapeutischen Behandlung von Krankheiten geeignet, die ausgewählt sind aus der Gruppe von Infektionskrankheiten, septischer Schock, Tumore, Krebs, Autoimmunerkrankungen, Allergien und chronische oder akute Entzündungsprozesse.

In einer bevorzugten Ausführungsform ist die Krebs- oder Tumorerkrankung, die behandelt, prophylaktisch verhindert oder deren Wiederauftreten verhindert wird, ausgewählt aus der Gruppe von Krebs- oder Tumorerkrankungen des Hals-Nasen-Ohren-Bereichs, Mediastinums, Gastrointestinaltraktes (einschließlich Kolonkarzinome, Magenkarzinome, Dickdarmkrebs, Dünndarmkrebs, Pankreaskarzinome, Leberkarzinome), Urogenitalsystems (einschließlich Nierenzellkarzinome), gynäkologischen Systems (einschließlich Ovarialkarzinome) und endokrinen Systems sowie der Lunge (einschließlich Lungenkrebs), Brust (einschließlich Mammakarzinome) und Haut sowie Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppression-bezogenen Malignitäten, multiplen Myelomen und Tumor-Metastasen.

Die Autoimmunerkrankungen sind vorzugsweise ausgewählt aus der Gruppe umfassend Arthritis, autoimmune Hepatitis, chronische Gastritis, Neurodermatitis, Schuppenflechte, Arthrose, rheumatische Erkrankungen, rheumatoide Arthritis, juvenile idiopathische Arthritis, Morbus Crohn, eiternde Dickdarmentzündung, Diabetes, entzündliche Darmerkrankungen, Multiple Sklerose und/oder allergische Entzündungen.

Die Nanopartikel werden auch zur Prophylaxe oder Therapie von Krankheiten eingesetzt, die durch Mikroorganismen hervorgerufen werden, die für Säuger pathogen sein können. Das heißt, dass sich die erfindungsgemäße Wirkung sowohl gegen Mikroorganismen richtet, die durch eine Störung des natürlichen Gleichgewichts der Mikroorganismenflora, die einen Wirtsorganismus besiedelt, und/oder bei immungeschwächten Wirten gesundheitsschädigende Abläufe zu ihrem eigenen Vorteil ausführen können, als auch gegen solche, die inhärent pathogen sind. Bevorzugte Mikroorganismen sind Viren, Bakterien, Pilze und/oder tierische Einzeller. Besonders bevorzugt sind Bakterien, wobei grampositive und gramnegative Bakterien in ihrem Wachstum beeinflusst werden. Beispiele für Krankheiten, die mit den Nanopartikeln zu behandeln sind, sind Hepatitis B, Hepatitis C, HIV, Herpes, Tuberkulose, Lepra oder Malaria, die durch vorgenannte Mikroorganismen hervorgerufen werden.

Dem Fachmann ist bekannt, dass die Induktion der T-Zell-Proliferation und/oder neutralisierender Antikörper zu fast jedem Zeitpunkt von Vorteil sein kann. Vorliegend werden die erfindungsgemäßen Nanopartikel und deren Dispersionen primär zur Immuntherapie eingesetzt, so dass eine Impfung im Sinne der Erfindung vorzugsweise eine Applikation des erfindungsgemäßen Impfstoffs nach Diagnose und/oder Ausbruch einer Krankheit ist, die auf eine Immuntherapie anspricht. Die Impfung sollte vorzugsweise zeitnah nach der Diagnose oder dem Ausbruch der Krankheit erfolgen und kann als Therapie auch mehrfach appliziert werden, um die anfängliche proliferative Immunantwort des Organismus durch mehrere Injektionen zu verstärken. Folglich wird auch die Verlaufskontrolle als Art der therapeutischen Behandlung verstanden, wenn die Nanopartikel in bestimmten Intervallen verabreicht werden, z.B. um die Symptome einer Krankheit vollständig zu beseitigen. In einer bevorzugten Ausführungsform der vorliegenden Erfindung werden die erfindungsgemäßen Nanopartikel bzw. Dispersionen daraus zur Therapie von Krebs und/oder Tumoren verwendet, besonders bevorzugt zur Krebstherapie.

Selbstverständlich ist es ebenso vorteilhaft möglich, dass es nach einer prophylaktischen Applikation im Organismus zur Entwicklung eines aktiven Impfschutzes kommt. Eine prophylaktische Immuntherapie ist insbesondere ratsam, wenn ein Individuum Veranlagungen für den Ausbruch der vorgenannten Krankheiten aufweist, wie z.B. eine familiäre Vorbelastung, einen Gendefekt oder eine kürzlich überstandene Krankheit.

Die Erfindung betrifft also auch die Verwendung der erfindungsgemäßen Nanopartikel bzw. Dispersion zur Immunprophylaxe oder Immuntherapie. Die Erfindung betrifft auch die Verwendung einer wirksamen Menge der erfindungsgemäßen Nanopartikel bzw. Dispersion zur Herstellung eines Vakzins zur Immunprophylaxe oder Immuntherapie. Bei beiden Gegenständen sind die zu behandelnden Krankheiten ausgewählt aus der Gruppe, die Infektionskrankheiten, septischer Schock, Tumore, Krebs, Autoimmunerkrankungen, Allergien und chronische oder akute Entzündungsprozesse umfasst. Das Vakzin wird insbesondere auf nicht-chemischem Wege hergestellt, indem der Wirkstoff zusammen mit mindestens einem festen, flüssigen und/oder halbflüssigen Träger- oder Hilfsstoff und ggf. in Kombination mit einem oder mehreren weiteren Wirkstoffen in eine geeignete Dosierungsform gebracht wird. Die vorherige Lehre der Erfindung und deren Ausführungsformen sind gültig und ohne Einschränkungen auf die medizinische Verwendung anwendbar, sofern es sinnvoll erscheint.

Die Erfindung bezieht sich auf die Verwendung der erfindungsgemäßen Nanopartikel bzw. deren Dispersionen als Impfstoff zur Zielsteuerung von Antigenen an Antigen-präsentierende Zellen,zur Aktivierung des Immunsystems, vorzugsweise zur Aktivierung des Komplementsystems. Die Zielsteuerung erfolgt vorzugsweise *ex vivo* oder *in vitro,* indem die Antigen-tragenden Nanopartikel Zellen, Zellkulturen, Geweben oder Organen verabreicht werden, die Antigen-präsentierende Zellen umfassen. Die ex *vivo*-Verwendung wird insbesondere bei tierischen Zellen angewandt, die aus einem tierischen Organismus stammen, der von einer Krankheit betroffen ist, die ausgewählt ist aus der Gruppe von Infektionskrankheiten, septischer Schock, Tumore, Krebs, Autoimmunerkrankungen, Allergien und chronischen oder akuten Entzündungsprozessen. Die ex vivo-behandelten Zellen können entweder für Folgeuntersuchungen weiter in Kultur gehalten oder in ein Tier überführt werden, wobei es sich um das Wirtstier oder ein anderes Tier handeln kann. Die erfindungsgemäße ex *vivo*-Zielsteuerung ist insbesondere von Vorteil, um den spezifischen Aufbau der Nanopartikel betreffs Partikelgröße, Antigen, Befestigung und Mehrfachfunktionalisierung zu testen, so dass unter Auswertung dieser ex *vivo*-Daten die *in vivo*-Dosis entsprechend voreingestellt werden kann. Im Ergebnis dessen wird der therapeutische Effekt in Form der erworbenen Immunantwort signifikant erhöht. Es ist ebenso möglich, die T-Zellen eines Patienten außerhalb des Körpers direkt mittels jener Antigen-präsentierenden Zellen, die den Nanopartikeln ausgesetzt waren, zu stimulieren und danach die T-Zellen für Forschungszwecke zu verwenden.

In einer bevorzugten Ausgestaltung der Erfindung werden die Antigene zu dendritischen Zellen geleitet. In einer besonders bevorzugten Ausgestaltung dieser Erfindung befinden sich die dendritischen Zellen in Lymphknoten. Es versteht sich, dass die letztgenannte Ausgestaltung zumindest ein Gewebe oder Organ voraussetzt, im besten Fall jedoch einen intakten tierischen Organismus. Es versteht sich ebenso, dass diese Voraussetzung für die Immun- oder speziell Komplementaktivierung erfüllt sein muss.

Die Nanopartikel können demnach *in vivo* verwendet werden, indem sie einem Tier über bekannte Routen direkt verabreicht werden, insbesondere einem Säuger, besonders bevorzugt einem Menschen. Die Nanopartikel können des Weiteren *ex vivo* eingesetzt werden, wobei die Antigen-präsentierenden Zellen zunächst aus einem Tier isoliert und anschließend mit den erfindungsgemäßen Nanopartikeln derart *ex vivo* behandelt werden, dass die Nanopartikel von den Zellen aufgenommen werden.

Ein Verfahren zur Induktion einer T-Zellantwort, Antikörperantwort und/oder dendritischen Zellreifung ist dadurch gekennzeichnet, dass die erfindungsgemäßen Nanopartikel, die als Dispersion und Impfstoff vorliegen, einem Säuger appliziert und die Proliferation von T-Zellen und/oder dendritischen Zellen und/oder die Bildung neutralisierender Antikörper induziert werden. Bevorzugte Organismen sind Mensch oder Tier. Durch die Offenbarung der erfindungsgemäßen Nanopartikel ist es dem Fachmann möglich, diese zur Induktion von T-Zellen und/oder neutralisierenden Antikörpern zu verwenden.

Dem Fachmann ist hierbei bekannt, dass er die erfindungsgemäßen Nanopartikel, die selbstverständlich als der erfindungsgemäße Impfstoff verwendet werden, in verschiedenen Dosierungen einem Organismus, insbesondere einem humanen Patienten, applizieren kann. Die Applikation sollte hierbei so erfolgen, dass eine möglichst große Menge von T-Zellen und/oder neutralisierenden Antikörpern generiert wird. Die Konzentration und die Art der Applikation kann vom Fachmann durch Routineversuche eruiert werden.

Das In-Kontakt-Bringen der erfindungsgemäßen Nanopartikel zur Verwendung als Impfstoff kann prophylaktisch oder therapeutisch erfolgen. Handelt es sich beispielsweise um eine prophylaktische Impfung zur Entwicklung eines aktiven Impfschutzes vor viralen Infektionskrankheiten, so soll die Infektion mit den Viren zumindest dergestalt verhindert werden, dass nach Eindringen einzelner Viren, beispielsweise in eine Wunde, eine weitere Vermehrung dieser stark vermindert wird oder dass eingedrungene Viren nahezu vollständig abgetötet werden. Bei einer therapeutischen Induktion einer Immunantwort liegt bereits eine Infektion des Patienten vor und die Induktion der T-Zellen und/oder neutralisierenden Antikörper erfolgt, um die bereits im Körper befindlichen Viren abzutöten bzw. in ihrer Vermehrung zu hemmen.

Sämtliche genannte sowie weitere Bestandteile bzw. Komponenten sind dem Fachmann geläufig und können in Routineversuchen eine spezielle Ausgestaltung für die erfindungsgemäße Lehre erfahren.

Im Rahmen der vorliegenden Erfindung wird also erstmalig ein ultrakleines Siliciumdioxid-Antigen-Konjugat bereitgestellt, das eine effektive zelluläre Immunantwort nach Vakzinierung fördert. Das Konjugat adressiert einen zweifachen Wirkmechanismus, indem es sowohl zur spezifischen Zielsteuerung an Antigen-präsentierende Zellen als auch zur gleichzeitigen Komplementaktivierung befähigt ist. Die Nanopartikel kleiner 50 nm besitzen im Vergleich zu großen Nanopartikeln des Stands der Technik eine vielfach höhere Zielsteuerungseffizienz. Infolge des effizienten Übergangs in die Lymphbahnen kann der biophysikalische Mechanismus des interstitiellen Flusses dahingehend vorteilhaft genutzt werden, dass dendritische Zellen der Lymphknoten angesteuert werden. Die Konvektion der Nanopartikel in diesem neuen Transportweg ermöglicht eine passive Zielsteuerung, infolgedessen sich ein aufwendiges zellspezifisches Targeting erübrigt, aber trotzdem besonders viele Zellen erreicht werden, da die dendritischen Zellen in Lymphknoten in großer Zahl vorliegen. Diese Eigenschaften bilden die Grundlage für eine zuverlässige Erkennung dendritischer Zellen der Lymphknoten - womit das Fehlen von Kreuzreaktivitäten (inkl. Ansteuern peripherer dendritischer Zellen) eingeschlossen ist - und eine reproduzierbare, zuverlässige und vollständige Phagozytose in diese Antigen-präsentierenden Zellen. Am angesteuerten Ziel kommt die zweite vorteilhafte Eigenschaft der Nanopartikel auf Siliciumdioxid-Basis zum Tragen, deren intrinsischer Adjuvanseffekt das Immunsystem und insbesondere das Komplementsystem aktiviert. Während die Stärke der Aktivierung unabhängig vom gewählten Antigen ist, kann sie über die Partikelgröße verändert werden. Das Fehlen zusätzlicher Hilfsstoffe und/oder Modifikationen der Nanopartikeloberfläche (z.B. Polyhydroxylierung) zur Aktivierung des Immun- bzw. Komplementsystems stellt eine deutliche Vereinfachung und Kostenersparnis dar.

Die erfindungsgemäßen Nanopartikel sind durch das anorganische, inerte und biokompatible Matrixmaterial charakterisiert, das insbesondere für die prophylaktische oder therapeutische Vakzinierung anwendbar ist. Die Entwicklung der hier vorgestellten Nanopartikel aus Siliciumdioxid-Antigen-Konjugat ist ebenfalls eine vielversprechende Strategie, um den therapeutischen Index von cytotoxischen Wirkstoffen zu verbessern. Insbesondere eine labile Verknüpfung der Bestandteile gewährleistet das Freisetzen des antigenen Therapeutikums in spezifischen Kompartimenten des Körpers, so dass eine Reduzierung möglicher Nebenwirkungen zu erwarten ist. Die Nanopartikel zeichnen sich auch durch eine hohe pharmazeutische Stabilität aus und sind nicht zuletzt aufgrund ihrer geringen Größe gut zu handhaben. Die ultrafeinen Nanodispersionen mit Partikeln von monodisperser Größe sind vorteilhaft zur Sterilfiltrierbarkeit geeignet.

Es versteht sich, dass diese Erfindung nicht auf die spezifischen Methoden, Partikel und Bedingungen beschränkt ist, wie sie hierin beschrieben sind, da solche Dinge variieren können. Es versteht sich des Weiteren, dass die vorliegend verwendete Terminologie ausschließlich dem Zweck der Beschreibung besonderer Ausführungsformen dient und nicht den Schutzumfang der Erfindung einschränken soll. Wie vorliegend in der Spezifikation einschließlich der anhängigen Ansprüche verwendet, schließen Wortformen im Singular, wie z. B. "ein", "eine", "einer", "der" oder "das" die Entsprechung im Plural ein, sofern der Kontext nicht eindeutig etwas anderes vorgibt. Beispielsweise enthält der Bezug auf "ein Antigen" ein einzelnes Antigen oder mehrere Antigene, die wiederum identisch oder verschieden sein können, oder der Bezug auf "ein Verfahren" schließt äquivalente Schritte und Verfahren ein, die dem Fachmann bekannt sind.

Im Folgenden wird die Erfindung anhand nicht limitierender Beispiele für konkrete Ausführungsformen näher erläutert. Die Beispiele sind insbesondere dahingehend zu interpretieren, dass sie nicht auf die konkret veranschaulichten Merkmalskombinationen beschränkt sind, sondern die beispielhaften Merkmale wiederum frei kombiniert werden können, solange die Aufgabe der Erfindung gelöst wird.

### Beispiel 1: Herstellung monodisperser Siliciumdioxid-Partikel

Die Herstellung der monodispersen Siliciumdioxid-Partikel erfolgte - wie in EP 0 216 278 B1 beschrieben - durch Hydrolyse von Tetraalkoxysilanen in wässrig-alkoholisch-ammonikalischem Medium, wobei man zunächst ein Sol von Primärteilchen erzeugt und anschließend durch ein kontinuierliches, nach Maßgabe des Abreagierens kontrolliertes Zudosieren von Tetraalkoxysilan die erhaltenen SiO₂-Partikel auf die gewünschte Teilchengröße bringt. Für die Herstellung von 50 g SiO₂-Partikeln mit einer Größe von 25 nm benötigt man beispielsweise 1,2 L EtOH als Lösungsvermittler, 860 mL deionisiertes Wasser, 167 mL Tetraethylorthosilicat (TEOS) und 28,5 mL 25%-iger wässriger Ammoniaklösung.

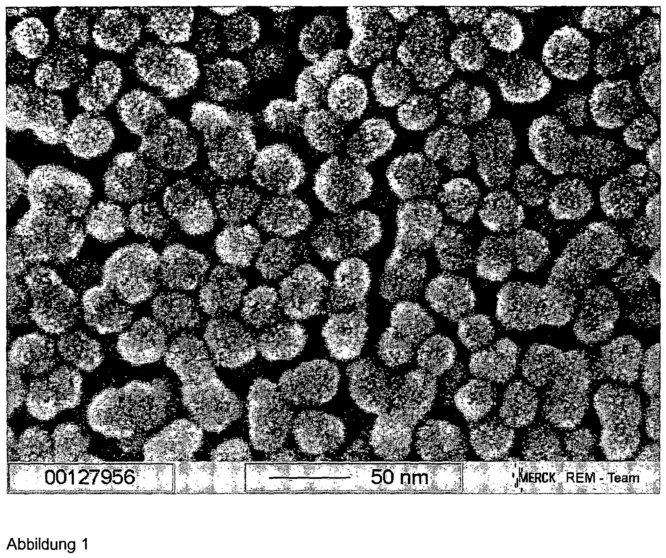

Die kugelförmigen Siliciumdioxid-Partikel wurden mittels dynamischen Lichtstreuungs-Messungen mit einem Zetasizer Nano ZS (Malvern Instruments, Herrenberg, Deutschland) bestimmt. Der Malvern-PDI (Polydispersitätsindex) mit Werten <0.1 zeigte eine monodisperse Verteilung. In Abbildung 1 ist die Partikelgröße und -morphologie mittels REM-Aufnahme dargestellt.

### Beispiel 2: Herstellung des OVA-Peptid-Fragments SIINFEKL mit N-terminaler Alkingruppe

Das Peptid wurde mittels Fmoc-Chemie auf einem Rink-Amid Harz aufgebaut. N-alpha-Fmoc-geschützte Aminosäuren mit geeigneten Seitenkettenschutzgruppen wurden eingesetzt. Als Lösungsmittel diente N-Methyl-Pyrrolidon. Zunächst wurde die Peptidkette in einem Syntheseautomaten (Applied Biosystems Modell ABI 433 A) aufgebaut. Nach Vervollständigung der Sequenz wurde die terminale Fmoc-Schutzgruppe abgespalten. Das Polymer wurde in einer Spritze manuell mit der Alkin-Carbonsäure gekoppelt. Es wurde sorgfältig mit DMF, gefolgt von Dichlormethan und Methanol gewaschen und das Harz über Nacht im Vakuum getrocknet. Zur Abspaltung und Entschützung wurde das Harz mit 5 ml eines Gemischs aus TFA/H2O/Phenol/Triisopropylsilan (37:1:1:1) versetzt und für 2 h bei Raumtemperatur geschüttelt. Die TFA-Lösung wurde in ein Zentrifugenglas überführt und durch langsame Zugabe von Diethylether bei 4 °C gefällt, abzentrifugiert, zwei Mal durch Zugabe von Diethylether gewaschen, getrocknet und in 2 ml H2O/Acetonitril (1:1 v/v) aufgenommen. Die Aufreinigung erfolgte durch RP-HPLC mit einer RP-select B-Säule (150 x 10 mm) mit einem Gradienten von 0% B - 100% B in 7.5 min (A = H2O und B = Acetonitril, beide mit 0.1% TFA versetzt), Flußrate = 10 mL/min. Die Homogenität und Identität des gereinigten Produkts wurde durch analytische HPLC und Massenspektrometrie bestätigt. Nach RP-HPLC-Aufreinigung wurde das Peptid lyophilisiert.

### Beispiel 3: Funktionalisierung der Siliciumdioxid-Partikel mit 3-Brompropyltrimethoxysilan

1 g der im Beispiel 1 hergestellten SiO₂-Partikel (25 nm) wurden in einem Ethanol-Wasser-Gemisch (100 mL; 4:1) suspendiert und mit 0,3 mL 25 %-iger wässriger Ammoniaklösung versetzt. Anschließend wurde 0,25 mL 3-Brompropyltrimethoxysilan (ABCR, Karlsruhe, Deutschland), gelöst in 10 mL Ethanol, über einen Tropftrichter langsam zugetropft und am Rückfluss für ca. 20 h erhitzt. Das Reaktionsgemisch wurde auf Raumtemperatur abgekühlt und die SiO₂-Partikel 5 x mit einem Ethanol-Wasser-Gemisch (4 : 1) gewaschen. Alle Waschschritte wurden mittels 10-minütiger Zentrifugation bei 9000 x g und 20°C in einer Temperaturkontrollierten Zentrifuge in 50 mL-Reaktionsgefäßen und mittels Resuspension der Partikel unter Verwendung eines Ultraschallfingers durchgeführt.

### Beispiel 4: Umsetzung der funktionalisierten Siliciumdioxid-Partikel mit Natriumazid

Die im dritten Beispiel mit 3-Brompropyltrimethoxysilan funktionalisierten SiO₂-Partikel wurden in 80 mL Dimethylsulfoxid (DMSO) umdispergiert, mit 1 g Natriumazid und 100 mg Tetrabutylammoniumbromid versetzt und bei 80°C für 40 Stunden gerührt. Anschließend wurden 200 mL deionisierten Wasser zugegeben und die Partikel mittels Ultrafiltrations-Verfahren unter Verwendung einer Membran mit einem Rückhaltevermögen von 10 kDa (Millipore, Bedford, USA) isoliert und mit 600 mL deionisiertem Wasser gewaschen.

### Beispiel 5: Verknüpfung des OVA-Peptid-Fragments SIINFEKL an die funktionaliserten SiO₂-Partikel

Die im vierten Beispiel hergestellten Azid-SiO₂-Partikel wurden in 40 mL Acetonitril resuspendiert, mit dem OVA-Peptid-Fragment aus Beispiel 1 (SIINFEKL-Alkin), Diisopropylethylamin (DIPEA) und Kupfer-(I)-iodid versetzt und bei Raumtemperatur für ca. 20 h gerührt. Die Suspension wurde mit 100 mL deionisiertem Wasser versetzt, mittles Ultrafiltration über eine 10 kDa Membran (Millipore, Bedford, USA) isoliert und mit 200 mL deionisiertem Wasser und 50 mL wässriger EDTA-Lösung gewaschen.

### Beispiel 6: Testung der Silikananopartikel auf In vivo-Adjuvanz-Aktivität unter Verwendung von PBL-Phänotypen (peripheral blood lymphocytes) als Read-out

Die Untersuchungen wurden an C57B1/6-Mäusen durchgeführt. Die Tiere wurden in 3 Gruppen unterteilt (2 Mäuse pro Gruppe), die entweder PBS (phosphate buffered saline), LPS (Lipopolysaccharid) oder Silikananopartikel (25nm) verabreicht bekamen. PBS diente als Kontrolle und LPS als Referenz (TLR4 Agonist) gegen die Silikananopartikel bezüglich ihrer Adjuvanz-Aktivität gebenchmarkt wurden.

Im Experiment wurden unmodifizierte Silikananopartikel von 25nm Größe untersucht, die nach dem in Beispiel 1 beschriebenen Verfahren hergestellt, dialysiert und anschließend steril filtriert wurden. Im Anschluss wurde die Silikadispersion auf Endotoxine untersucht, um sicherzustellen, dass der Read-out des Tierexperiments nicht durch Endotoxin-Kontamination der Nanopartikel verfälscht würde. Die Endotoxinkonzentration in der untersuchten Nanopartikeldispersion liegt unterhalb des von der Ph.Eur. empfohlenen Maximallevels für flüssige, parenterale Formulierungen von 0.5 IU/ml.

Jeweils 100µl der Testlösungen bzw. -dispersion wurde den Tieren s.c. (subcutan) in die Flanke verabreicht. Die Nanopartikeldispersion enthielt 450µg Silikananopartikel in 100µl PBS. Als Referenz wurde 10µg/Maus LPS appliziert.

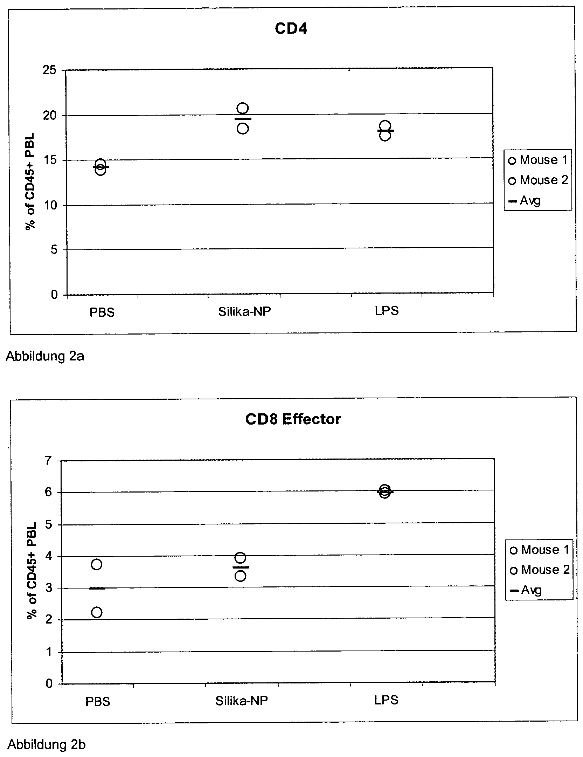

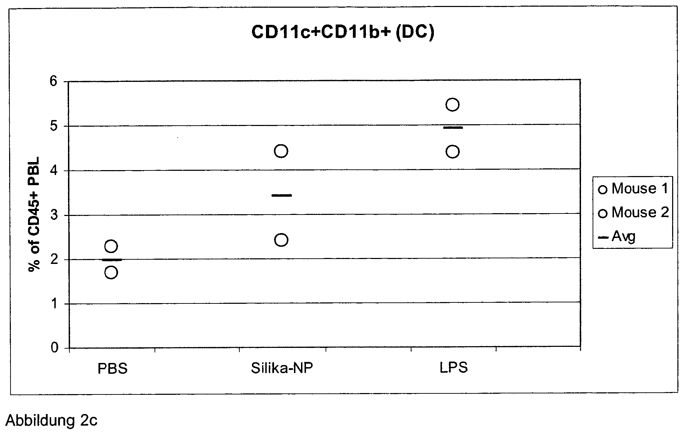

75-100µl peripheres Blut/Maus wurde durch retro-orbitale Blutung mittels heparinisierten Kapillarröhrchen entnommen und in heparinisierten Eppendorfcups gesammelt. Die Blutproben wurden mit verschiedenen Detektions-Cocktails markiert. Im Anschluss wurde mittels FACS (fluorescence activated cell sorting) die prozentuelle Verteilung immunologisch relevanter PBL-Phänotypen bestimmt. In Abbildungen 2a-c ist für die vier PBL-Subpopulationen CD4 (Abb. 2a), CD8 Effektor (Abb. 2b), CD11b⁺ und CD11c⁺(DC) (Abb. 2c) ihr prozentueller Anteil an der PBL-Gesamtpopulation aufgetragen. Die Daten belegen, dass nach Gabe der Silikananopartikel die Anzahl immunologisch bedeutsamer T-Zellen und dentritischer Zellen im Vergleich zur Vehikelkontrolle PBS erhöht wurde, was auf einen adjuvanten Effekt der Silikananopartikel hindeutet.

### Beispiel 7: ^{99m}Tc-Markierung von Silikananopartikeln in Wasser

Die Nanopartikellösung (25nm, Feststoffgehalt 9,0 mg/ml) wurde vor Gebrauch durch ein MILEX-GV 0,22 µm Filter Unit filtriert. Zu ^{99m}Tc (132 MBq in 40 µl) wurden 50 µl Silikananopartikel gegeben und die Lösung gemischt. Dann wurden 2 µl einer SnCl₂ Lösung (0.1% SnCl₂ Dihydrat in 10 mM HCl) zugegeben und die Lösung erneut gemischt. Nach ca. 2 min wurden 150 µl 0,5 M Phosphatpuffer pH 8 zugegeben und die Lösung in ein Millipore Microcon Ultracel YM-100 Centrifugal Filter Device überführt und 3 min bei 13000 rpm zentrifugiert. Der Filter wurde zweimal mit je 200 µl 0,5 M Phosphatpuffer pH 8 gewaschen. Das Gesamtfiltrat enthielt 46.84 MBq ^{99m}Tc. Im Filter blieben 69,8 BMq ^{99m}Tc. Die Partikel im Filter wurden zweimal in je 200 µl 0,5 M Phosphatpuffer pH 8 suspendiert und durch Umdrehen des Filters und kurzes Zentrifugieren zurückgewonnen. Es wurde eine mit 28 MBq ^{99m}Tc-markierte Partikelsuspension erhalten. Die erhaltene Partikelsuspension wurde anschließend für das Tierexperiment verwendet.

### Beispiel 8: In vivo Bildgebung der ^{99m}Tc-markierten Silikananopartikel

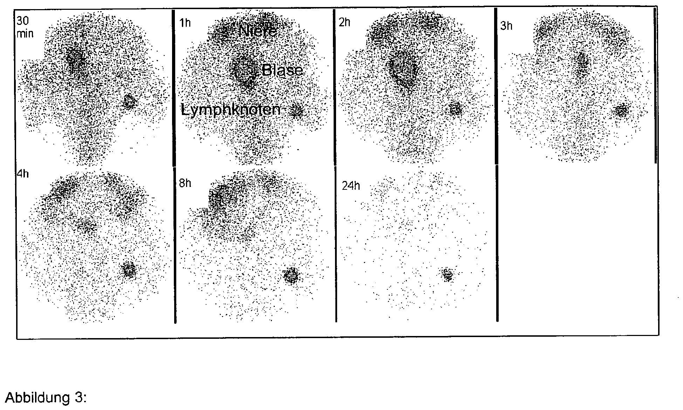

Die Partikel wurden wie in Beispiel 7 beschrieben markiert. Anschließend erfolgte die nicht-invasive Bildgebung der Wanderung in den Sentinel-Lymphknoten. Zu diesem Zweck wurden ca. 400-500 g schwere weibliche Wistar-Ratten mittels Inhalationsnarkose mit Isofluoran narkotisiert. Die Tiere erhielten eine subkutane Injektion einer 10-20 MBq ^{99m}Tc enthaltenden klaren Suspension der radiomarkierten Partikel in eine der beiden Hinterpfoten. Anschließend wurden die Tiere, weiter unter Narkose, mittels Szintigraphie untersucht. Zu diesem Zweck wurde die Anreicherung im Unterkörper der Tiere zu verschiedenen Zeitpunkten mit einer Gamma-Kamera dargestellt. Die Kinetik dieser Aufnahmen zeigt eine deutliche Anreicherung der Partikel in den von der Hinterpfote ableitenden Lymphknoten (siehe Abbildung 3). Hierbei handelt es sich um den Sentinel-Lymphknoten. Zudem zeigt sich eine transiente Anreicherung in den Nieren und der Blase. Die Anreicherung im Lymphknoten ist hingegen über 24h anhaltend. Es konnten Wanderungsraten von ca. 1% der applizierten Dosis beobachtet werden. Als Kontrollversuch erfolgte die Analyse der Wanderung von ^{99m}Tc-markiertem Nano-Albumon, einer kommerziell erhältlichen Kolloid-Präparation zur Darstellung des Sentinel-Lympknotens. Die Anreicherung dieser Substanz war vergleichbar mit der der Silica-Partikel. Durch ein weiteres Kontrollexperiment, in dem freies ^{99m}Tc durch eine subkutane Injektion in eine der beiden Hinterpfoten von weiblichen Wistar-Ratten gegeben wurde, konnte sichergestellt werden, dass freies ^{99m}Tc sich nicht im Lymphknoten anreichert.

Damit konnte gezeigt werden, dass die Silikananopartikel die Lymphknoten targetieren können, worüber eine Aktivierung des Immunsystems erfolgen kann.

Die nachfolgenden Beispiele betreffen pharmazeutische Zubereitungen.

### Beispiel A: Injektionsgläser

Eine Lösung von 100 g Nanopartikeln und 5 g Dinatriumhydrogenphosphat wird in 3 I zweifach destilliertem Wasser mit 2 N Salzsäure auf pH 6,8 eingestellt, steril filtriert, in Injektionsgläser abgefüllt, unter sterilen Bedingungen lyophilisiert und steril verschlossen. Jedes Injektionsglas enthält 5 mg Nanopartikel.

### Beispiel B: Suppositorien

Man schmilzt ein Gemisch von 20 g Nanopartikeln mit 100 g Sojalecithin und 1400 g Kakaobutter, gießt in Formen und lässt erkalten. Jedes Suppositorium enthält 20 mg Nanopartikel.

### Beispiel C: Lösung

Man bereitet eine Lösung aus 1 g Nanopartikeln, 9,38 g NaH₂PO₄ *2 H₂O, 28,48 g Na₂HPO₄ *12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg Nanopartikel mit 99,5 g Vaseline unter aseptischen Bedingungen.

### Beispiel E: Tabletten

Ein Gemisch von 1 kg Nanopartikeln, 4 kg Lactose, 1,2 kg Kartoffelstärke, 0,2 kg Talk und 0,1 kg Magnesiumstearat wird in üblicher Weise zu Tabletten verpresst, derart, dass jede Tablette 10 mg Nanopartikel enthält.

### Beispiel F: Dragees

Analog Beispiel E werden Tabletten gepresst, die anschließend in üblicher Weise mit einem Überzug aus Saccharose, Kartoffelstärke, Talk, Tragant und Farbstoff überzogen werden.

### Beispiel G: Kapseln

2 kg Nanopartikel werden in üblicher Weise in Hartgelatinekapseln gefüllt, so dass jede Kapsel 20 mg der Nanopartikel enthält.

### Beispiel H: Ampullen

Eine Lösung von 1 kg Nanopartikel in 60 I zweifach destilliertem Wasser wird steril filtriert, in Ampullen abgefüllt, unter sterilen Bedingungen lyophilisiert und steril verschlossen. Jede Ampulle enthält 10 mg Nanopartikel.

### Beispiel I: Inhalations-Spray

Man löst 14 g Nanopartikel in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Monodisperse Nanopartikel umfassend eine Matrix aus mehr als 50 % Siliciumdioxid, die an ihrer Oberfläche eine oder mehrere chemische Gruppen als Linker zur Befestigung und kontrollierten Freisetzung eines Antigens aufweisen, wobei an den Linker mindestens ein Antigen ortsspezifisch und kovalent gebunden ist, wobei die Partikel eine Größe von 5 bis 50 nm haben, von der die Standardabweichung maximal 15 % beträgt, zur Verwendung als Impfstoff zur Zielsteuerung von Antigenen an Antigen-präsentierende Zellen, zur Aktivierung des Immunsystems und als Adjuvans.

2. Nanopartikel zur Verwendung nach Anspruch 1, wobei die Nanopartikel keine weiteren Adjuvantien umfassen.

3. Nanopartikel zur Verwendung nach Anspruch 1 oder 2, wobei der Linker ein Alkoxysilan ist.

4. Nanopartikel zur Verwendung nach einem der Ansprüche 1 bis 3 zur Zielsteuerung der Antigene an dendritische Zellen in Lymphknoten.

5. Nanopartikel zur Verwendung nach einem der Ansprüche 1 bis 4 zur Aktivierung des Komplementsystems.

## Claims

1. Monodisperse nanoparticles comprising a matrix of more than 50% of silicon dioxide which have one or more chemical groups on their surface as linker for the attachment and controlled release of an antigen, where at least one antigen is location-specifically and covalently bonded to the linker, where the particles have a size of 5 to 50 nm, of which the standard deviation is at most 15%, for use as vaccine for the targeting of antigens at antigen-presenting cells, for the activation of the immune system and as adjuvant.

2. Nanoparticles for use according to Claim 1, where the nanoparticles comprise no further adjuvants.

3. Nanoparticles for use according to Claim 1 or 2, where the linker is an alkoxysilane.

4. Nanoparticles for use according to one of Claims 1 to 3 for targeting the antigens at dendritic cells in lymph nodes.

5. Nanoparticles for use according to one of Claims 1 to 4 for activation of the complement system.

## Revendications

1. Nanoparticules monodispersées comprenant une matrice de plus de 50% de dioxyde de silicium qui possèdent un ou plusieurs groupements chimiques à leur surface comme bras de liaison pour la fixation et la libération contrôlée d'un antigène, où au moins un antigène est fixé au bras de liaison de manière covalente et spécifique d'un emplacement, où les particules possèdent une taille allant de 5 à 50 nm, dont l'écart-type est d'au plus 15%, pour une utilisation comme vaccin pour le ciblage d'antigènes au niveau de cellules présentatrices d'antigène, pour l'activation du système immunitaire et comme adjuvant.

2. Nanoparticules pour une utilisation selon la revendication 1, où les nanoparticules ne comprennent pas d'autres adjuvants.

3. Nanoparticules pour une utilisation selon la revendication 1 ou 2, où le bras de liaison est un alcoxysilane.

4. Nanoparticules pour une utilisation selon l'une des revendications 1 à 3, pour le ciblage des antigènes au niveau de cellules dendritiques dans les noeuds lymphoïdes.

5. Nanoparticules pour une utilisation selon l'une des revendications 1 à 4, pour l'activation du système du complément.
